(19) Europäisches Patentamt
European Patent Office
Office européen des brevets

(11) Publication number: **0 513 470 A2**

# (12) EUROPEAN PATENT APPLICATION

(21) Application number: **91870088.1**

(51) Int. Cl.$^5$: **C07C 69/44**, C08K 5/11

(22) Date of filing: **03.06.91**

(30) Priority: **13.05.91 US 697574**

(43) Date of publication of application:
**19.11.92 Bulletin 92/47**

(84) Designated Contracting States:
**AT BE CH DE DK ES FR GB GR IT LI LU NL SE**

(71) Applicant: **MONSANTO COMPANY**
**Patent Department 800 North Lindbergh Boulevard**
**St. Louis, Missouri 63167(US)**

(72) Inventor: **David, Donald Joseph**
**11 Indian Pipe Lane**
**Amherst, Massachusetts 01002(US)**
Inventor: **Fariss, Robert Hardy**
**51 Westhampton Road**
**Northampton, Massachusetts 01060(US)**
Inventor: **Knowles, Daniels Carl**
**96 Allen Road**
**Belchertown, Massachusetts 01007(US)**
Inventor: **Tetreault, Roland Joseph**
**71 Hilltop Street**
**Springfield, Massachusetts 01128(US)**

(74) Representative: **Ernst, Hubert et al**
**Monsanto Services International S.A., Patent Department, Avenue de Tervuren 270-272, Letter Box No. 21**
**B-1150 Brussels(BE)**

## (54) Adipate compositions.

(57) A composition of matter comprising a mixture of pure and mixed adipates made from: (a) n-hexanol and (b) cyclohexanol wherein the proportions of (a) and (b) in parts by weight per 100 parts of (a) and (b) are: (a) 50-70 (b) 50-30.

EP 0 513 470 A2

## BACKGROUND OF THE INVENTION

This invention relates to adipate compositions and more particularly to such compositions as plasticizers for polyvinyl butyral resin.

Polyvinyl butyral (PVB) resin as sheet for use as optically transparent, impact-dissipating interlayer in multilayered laminated safety glazings is well known. Since the glass transition temperature of unplasticized PVB resin is too high for it to be a useful elastomer for impact dissipation in these applications, it is also known to reduce such temperature to a useful range by incorporating a plasticizer in such resin.

In choosing a plasticizer for PVB resin for such applications, it is further known to balance the impact-dissipating and edge stability capabilities of a glazing interlayer by using a mixture where each component of the mixture is better than the other in promoting one of these properties. Representative mixtures and their optimization are disclosed in U.S. Nos. 4,243,572 and 4,371,586 where aryl adipates in a mixture provide edge stability to the interlayer. Light stability of laminates using plasticized PVB resin, however, is also required for the intended architectural and motor-vehicle window applications involving prolonged exposure to sunlight. Optimizing shock absorbing, edge-delamination-resistant and UV stability properties in an interlayer through choice of an appropriate plasticizer has, to the best of present knowledge, not been accomplished in the prior art.

## SUMMARY OF THE INVENTION

Now improvements have been made to adipate compositions which mitigate shortcomings of the prior art.

Accordingly, the principal object of this invention is to provide an improved adipate composition.

Another object is to provide such a composition which is suitable as a plasticizer for PVB resin.

A specific object is to provide such a composition which, when present with PVB resin as an interlayer in a laminated glazing, promotes color stability to UV light and improved resistance to edge delamination without suffering any penalty in low temperature impact performance.

Other objects of this invention will in part be obvious and will in part appear from the following description and claims.

These and other objects are accomplished by providing a composition of matter comprising a mixture of pure and mixed adipates made from a mixture of (a) n-hexanol and (b) cyclohexanol wherein the proportions of (a) and (b) in parts by weight per 100 parts of (a) and (b) are: (a) 50-70, preferably 60-70 (b) 50-30, preferably 40-30.

## BRIEF DESCRIPTION OF THE DRAWINGS

In describing the overall invention, reference will be made to the accompanying drawings wherein:

Fig. 1 a is graph illustrating the simulated edge stability improvement provided by compositions of the invention;

Fig. 2 is a phase diagram for the species of adipate ester composition of the invention; and

Fig. 3 is a plot for the species of Fig. 2 illustrating alcohol compositions which, when completely esterified, form the amounts shown on the y-axis of the specific ester components.

## DETAILED DESCRIPTION OF THE INVENTION

The adipate plasticizer compositions of the invention are prepared by esterifying adipic acid and an alcohol mixture of n-hexanol and cyclohexanol. The resulting product is a mixture of pure and mixed adipates or, more particularly, a mixture of di(n-hexyl) adipate, di(cyclohexyl) adipate and hexyl, cyclohexyl adipate, a pure adipate having the same alcohol moiety on each end of the molecule and a mixed adipate having a different alcohol moiety on each such end.

The structural formulas of pure and mixed adipates of the invention are as follows:

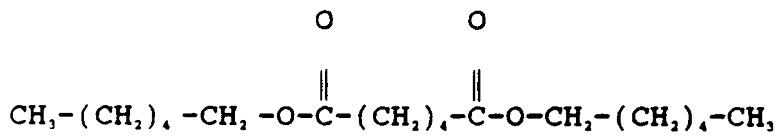

di(n-hexyl) adipate - pure adipate

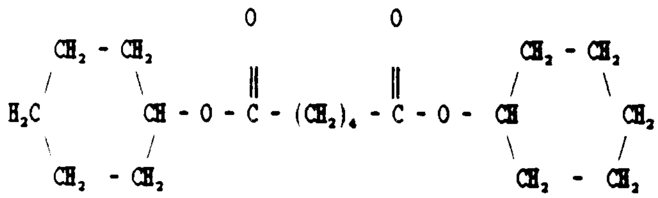

di(cyclohexyl) adipate - pure adipate

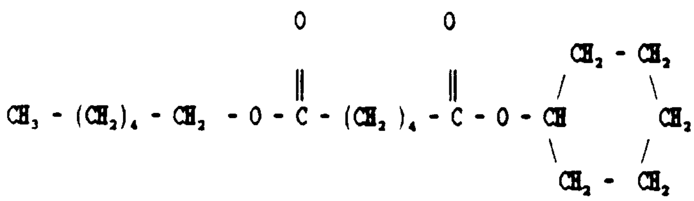

n-hexyl, cyclohexyl adipate - mixed adipate

The proportion of (a) and (b) alcohols chosen depends on i) the relative effect of the adipates formed by each alcohol on edge stability and impact performance of interlayer containing the particular adipate and ii) on avoiding haziness in the sheet from ester components of the plasticizer crystallizing with temperature. Proportions of 50-70, preferably 60-70, weight parts (a) and 50-30, preferably 40-30, weight parts (b) per 100 weight parts (a) and (b) generally provide an operable balance of properties. 65/35 is particularly preferred.

The PVB resin employed has a weight average molecular weight greater than 100,000, preferably from about 150,000 to 250,000, as measured by size exclusion chromatography using low angle laser light scattering. Such PVB comprises, on a weight basis, 15 to 25%, preferably 18 to 22% hydroxyl groups calculated as polyvinyl alcohol (PVOH); 0 to 10%, preferably 0 to 3% residual ester groups, calculated as polyvinyl ester, e.g. acetate, with the balance being butyraldehyde acetal. The amount of plasticizer in the formulation to form the interlayer sheet depends on the specific PVB resin used and the properties desired in the application. Generally between 15 to 50, preferably 25 to 40 parts plasticizer per 100 parts of PVB resin (PHR) is used.

PVB resin is produced by known aqueous or solvent acetalization processes wherein PVOH is reacted with butyraldehyde in the presence of acid catalyst to produce PVB, followed by neutralization of the catalyst, separation, stabilization and drying of the PVB resin. It is commercially available from Monsanto Company as Butvar® resin.

Plasticized PVB as interlayer sheet having a thickness of about 0.13 to 1.3 mm is formed by initially mixing the PVB resin with the plasticizer (and optional additional well-known property-enhancing additives) and then extruding the formulation through a sheeting die, i.e. forcing molten, plasticized PVB through a horizontally long vertically narrow die opening substantially conforming in length and width to that of the sheet being formed, or by casting molten polymer issuing from an extrusion die onto a specially prepared surface of a die roll in close proximity to the die exit to impart desired surface characteristics to one side of the molten polymer. When the roll surface has minute peaks and valleys, sheet formed of polymer cast thereon will have a rough surface on the side contacting the roll which generally conforms respectively to such valleys and peaks. A rough surface on the other side can be provided by the design of the die opening through which the extrudate passes. Such a die opening configuration is more particularly shown in Fig. 4 of U.S. No. 4,281,980. Alternative known techniques of producing a rough surface on one or both sides of an extruding sheet involve the specification and control of one or more of the following: polymer molecular weight distribution, water content and temperature of the melt. Such techniques are disclosed in

U.S. Nos. 2,904,844; 2,909,810; 3,994,654; 4,575,540 and European Patent No. 0185,863. As is known, this rough surface on the interlayer is only temporary and particularly functions to facilitate deairing during laminating after which it is melted smooth from the elevated temperature and pressure associated with autoclaving.

In addition to plasticizer, interlayers described herein may contain other additives such as dies, pigments, ultraviolet light stabilizers, antioxidants, adhesion control salts and the like.

The following tests were used to obtain results listed in the Examples.

A) Compatibility

1) Exudation Test - Plasticizer and PVB resin compatibility as reflected in long term interlayer edge stability in a laminate is simulated by this laboratory test. Various amounts of plasticizer and PVB resin were blended for 7 min at 150° C in a Brabender mixer equipped with sigma blades turning at 50 rpm. Using a heated hydraulic press (149° C, 5.5 MPa for 5 min), the resulting plasticized PVB resin was pressed into 0.76 mm thick sheets which are representative of interlayer usable with glass in laminated glazings. The sheets were cut into 17.5 x 38 mm samples, dried for 5 days in a desiccator and weighed to get dry weight. These samples were then placed in a wet desiccator (enclosed chamber containing water) for seven days. Each sample was then sandwiched between layers of absorbing cardboard, placed between jaws of a clamp being forced together at a pressure of 1.6 MPa. The clamped samples were placed in a wet desiccator for 10 days. The clamps were removed, the samples washed with warm water to remove residual cardboard, dried five days in a dry desiccator and again weighed. The weight difference in $gm/m^2$ of surface area is plasticizer exudation weight loss. Exudation Ratio is the ratio of the exudation weight loss of plasticizers according to the invention to that of 100% di-n-hexyl adipate (DHA) a control plasticizer which was run in parallel with invention plasticizers.

2) Edge Stability - 0.76 mm thick sheets of plasticized PVB resin were placed between two 15.2 cm x 15.2 cm x 0.23 cm glass plates. The layers were then held in an autoclave for about 7 min at 1.2 MPa, 135° C to laminate the glass and sheet. The laminates were then exposed in Florida at a 45 degree angle facing south and periodically visually observed for signs of edge delamination and compared to a control. Performance with exposure time was measured by Edge Stability Number (ESN). The ESN for a particular interlayer is based on nine sample laminates containing such interlayer. ESN is a value obtained by an arbitrary method for calculating edge defects in a laminate which is proportional to the amount of total delamination attributable to a particular test interlayer for the nine laminate samples of that interlayer. More particularly, it is a mathematical summation of defect length times a weighting factor corresponding to the depth or distance of each defect from the edge of the laminate sample.

B) Impact Resistance

1) Mean Penetration Velocity - 30.5 x 30.5 cm x 0.76 mm two ply glass laminates were prepared using the laminating conditions recited above and were individually horizontally positioned in a support frame. While at a constant laminate temperature, a 2.27 kg spherical ball was dropped from a designated height onto the center of the laminate. Two spaced magnetic coils were positioned beneath the test laminate. After penetrating a laminate, the ball sequentially passed through magnetic fields created by the coils and as these fields were disturbed the top coil triggered a timer "on" switch while the bottom coil turned it off. Knowing the time to traverse the distance between coils permits calculating ball velocity. This residual ball velocity is related to energy absorbed by the laminate and absorbed energy in miles per hour (mph) equals laminate Mean Penetration Velocity (MPV). Measured MPV is the average of multiple ball drops from different heights. MPV Ratio is the measured MPV for the experimental sample divided by the MPV of the control.

2) Pummel Adhesion measures interlayer adhesion to glass. Two ply glass laminates prepared as recited above for the MPV test were conditioned to -17° C and manually pummeled with a 1 pound (454g) hammer to break the glass. All broken glass unadhered to the PVB layer was then removed. The amount of glass left adhered to the interlayer is visually compared with a set of standards of known pummel scale, the higher the number of the standard, the more glass remaining adhered to the interlayer - i.e. at a pummel of Zero, no glass at all is left whereas at a pummel of 10, 100% of the interlayer surface is adhered to the glass. Desirable impact dissipation occurs at a pummel adhesion value of 3 to 7, preferably 4 to 6. At less than 3 too much glass is lost whereas at more than 7 adhesion is generally too high and shock absorption is poor.

C) Volatility

of plasticizer was determined by thermal gravimetric analysis (TGA) using a Perkin Elmer TGA7 in a nitrogen atmosphere over a 35-200 °C cycle at a 10 °C/min rate. Result as Volatility Ratio is the ratio of weight loss for the sample to that of the control.

D) Laminate Color

Yellowness Index - Two ply glass laminates with sample interlayer of 3.2 mm thick were prepared as recited above. Yellowness Index was measured using a Hunter D54 Spectrophotometer.

The following identifies abbreviations used for various adipate species in the Examples and Figs. 2 and 3.

| | | |
|---|---|---|
| DHA | di(n-hexyl) adipate | (pure) |
| HCHA | n-hexyl, cyclohexyl adipate | (mixed) |
| DCHA | di(cyclohexyl) adipate | (pure) |

The invention is further described with reference to the following Examples which are not intended to limit or restrict the invention. Unless otherwise indicated, all quantities and percentages are expressed in weight.

EXAMPLE 1 AND COMPARATIVE EXAMPLES C1, C2

Example 1 illustrates the improved combination of impact and edge stability performance of glass laminates made using interlayer formed from plasticizer mixtures according to the invention in comparison with Comparative Example C1 where the plasticizer was pure di(n-hexyl) adipate. Such latter control interlayer is considered an adipate standard for comparison purposes and is further described in U.S. No. 3,854,865, Examples 6-24. Comparative Example C2 illustrates that pure adipates formed using cyclohexanol alone are unsuitable.

Preparation of Adipate Mixtures

1) N-hexyl, cyclohexyl adipate (HCHA) The following was charged to a jacketed reactor equipped with an agitator and a reflux condenser having an oil-water separator on the downstream process side.

| Component | Amount (Kg) |
|---|---|
| Adipic Acid | 66.3 |
| N-hexanol | 60.3 |
| Cyclohexanol | 59.7 |
| Toluene | 36.0 |
| Methane Sulfonic Acid | 0.23 |

The agitator was started and the reactor contents heated at atmospheric pressure to about 110 °C to initiate reflux. Heating continued until the reaction mixture reached about 125 °C and for about four hours thereafter while removing water through the separator. Reactor pressure during this time was reduced to maintain this temperature. The esterification reaction was considered complete when water no longer issued from the separator.

To neutralize catalyst and unreacted adipic acid, 10% aqueous caustic was added in amount adequate to maintain a pH of 8 in the aqueous phase which was then decanted to remove sodium salts of the acids. The reactor contents was then washed twice with water with agitation. After phase separation the water phase was decanted and discarded. The pressure was reduced to 28 in (71.1 cm) mercury vacuum while heating to maintain 125 °C to remove toluene and unreacted alcohols and then sparged for about 30 min with steam at 3.15 $kg/cm^2$ absolute. The reaction mixture was then cooled and analyzed using gas chromatography as:
25% di(n-hexyl) adipate
25% di(cyclohexyl) adipate

50% n-hexyl, cyclohexyl adipate

Preparation and Testing of Interlayer

PVB resin for admixture with the foregoing adipate plasticizer mixture had less than 3% residual acetate groups and a PVOH content of 18.2 percent. Interlayer performance results obtained are in Table 1 following. Exudation Test results at various loadings of the plasticizers of Examples 1 and C1 are depicted in the drawing.

Table 1

| | Example 1 | Example C1 | Example C2 |
|---|---|---|---|
| Plasticizer | Mixture of DHA; HCHA;DCHA | DHA | DCHA |
| PHR | 32.8 | 32.0 | 35.7 |
| Volatility Ratio (VR) | 0.96 | 1.00 | 0.70 |
| Exudation Ratio | 0.41 | 1.0 | 0.13 |
| Pummel Adhesion (at -17.8°C and 0.5% $H_2O$) | 7.1 | 6.3 | 7.9 |
| Laminate Color (Yellowness Index) | 6.3 | 6.6 | ---- |
| Edge Stability No. (ESN)@ 26 months | 170 | 2156 | 79 |
| Mean Penetration Velocity Ratio | | | |
| -17.8°C | 0.63 | 1.00 | |
| 15.6°C | 1.06 | 1.00 | |
| 48.9°C | 1.17 | 1.00 | |

With respect to MPV Ratio for Example C2, to indirectly characterize the projected low temperature impact performance, the beta transition area from a dynamic mechanical test was considered. This area has been correlated with low temperature impact performance of plastics - see J. Heijboer, Journal of polymer Science; Part C; 16,3755 (1968). This beta transition area is predicted to be very small compared to that for control Example C1. This shows that though edge stability performance using the saturated ring adipate composition above is excellent, without the alkyl moiety in the adipate the important impact performance property is not achieved.

The above results of Ex. 1 vis-a-vis the DHA of control C1 illustrate: 1) significantly improved edge stability as reflected in the greatly reduced Exudation Ratios and ESN of (Ex. 1) and ii) somewhat inferior low temperature impact (pummel adhesion and MPV Ratio at -17.8° C) iii) somewhat lower VR. Low VR is desirable to minimize plasticizer loss from the interlayer, particularly with air autoclave laminating systems where elevated temperature and negative vacuum pressure conditions occur. Increasing the n-hexyl content in the composition (note the preferred alcohol range of 65/35 n-hexanol/cyclohexanol) will improve the low temperature -17.8° C MPV ratio.

EXAMPLE 2

This Example illustrates the effect of the concentration of components of an adipate ester composition of the type prepared in Example 1 (i.e. from n-hexanol and cyclohexanol) on the tendency to develop haze in PVB sheet containing such an ester mixture at temperatures usually encountered during sheet handling and storage before use in a glass laminate. More particularly, the effect of the concentration of DCHA (di-(cyclohexyl)adipate) in the mixture on such haze development is examined.

Fig. 2 is a solid-liquid phase diagram for DHA (di-n-hexyl adipate) and DCHA components of adipate

ester compositions prepared from mixtures of n-hexanol and cyclohexanol. In such figure, "S" and "L" are abbreviations for solid and liquid; %DHA (not directly shown) is 100 minus %DCHA.

Fig. 3 shows the weight fraction of an ester composition by ester species which will form after esterification with adipic acid versus the weight fraction of n-hexanol (weight fraction of cyclohexanol = 1 minus weight fraction n-hexanol) reacted to form such composition. This Figure is based on actual data and calculations - i.e. runs were made and esters analyzed at 0.25, 0.5 and 0.75 weight fraction n-hexanol reacted; points intermediate this data were calculated.

Referring to Fig. 3, completely esterifying a 30/70 n-hexanol/cyclohexanol (NHA/CHA) mixture produces an ester composition of about 42% DCHA, 12% DHA and 45% HCHA (n-hexyl, cyclohexyl adipate). From Fig. 2, at 42% DCHA (58% DHA by difference) solidification commences at about 20°C (68°F). This means a PVB sheet containing as plasticizer an effective amount of a composition containing 42% DCHA at temperatures less than about 20°C will undesirably appear milky or white from solidification of components of the adipate ester composition. Except where ambient temperature always exceeds about 20°C, this condition is considered unacceptable, since PVB sheet containing such a mixture could typically be exposed to 20°C during handling, for example, in northern regions of the U.S.A. On the other hand, an 80/20 NHA/CHA mixture (Fig. 3) will yield a composition of 3% DCHA, 70% DHA and 27% HCHA. From Fig. 2, at 3% DCHA solidification occurs at about minus 10°C so that above this temperature such composition is liquid and when admixed into PVB sheet will not present an appearance problem since minus 10°C should not be encountered during sheet handling. However, due to the dearth of cyclohexyl groups, such low level of DCHA would not be expected to provide optimum improved edge stability in PVB sheet containing such a plasticizer composition.

According to this Example, at 50/50 NHA/CHA, about 20% DCHA is formed on esterification (Fig. 3). From Fig. 2, solidification of a composition containing such a level of DCHA commences at about minus 5°C which is considered acceptable since sheet handling temperatures should seldom, if ever, get so low. At 70/30 NHA/CHA, 8% DCHA will be formed for which composition solidification commences at about minus 11°C. Between minus 11°C and minus 35°C a mixture of solid and liquid exists in thermodynamic equilibrium (the zone marked "S & L" in Fig. 2). Such a 70/30 NHA/CHA mixture and the adipate ester composition formed therefrom when used as a plasticizer in PVB sheet is considered to provide an acceptable balance of sheet performance properties without generating haziness in sheet during handling.

From the foregoing, a preferred composition is 60-70 parts n-hexanol, 40-30 parts cyclohexanol with 65/35 NHA/CHA being most preferred.

Using the apparatus described in Example 1, an esterification procedure similar to Example 1 was repeated. The charge to the reactor was:

| Component | Amount (Kg) |
|---|---|
| Adipic Acid | 102.6 |
| N-hexanol | 99.9 |
| Cyclohexanol | 59.9 |
| Stannous Oxide | 0.09 |

The agitator was started and the contents heated at atmospheric pressure to about 140°C to initiate reflux. Heating continued until the reaction mixture reached about 205°C and for about three hours thereafter while removing water through the separator. Reactor pressure once reaching 205°C was reduced to maintain reflux at 205°C. The esterification reaction was considered complete when water no longer issued from the separator.

To neutralize any unreacted adipic acid, 10% aqueous caustic was added at 95°C in amount adequate to maintain a PH of 8 in the aqueous phase which was then decanted to remove sodium salts of the acids. The reactor contents was then washed twice with water with agitation. After phase separation the water was removed and the oil phase was reduced to 28 in (71.1 cm) mercury vacuum while heating to maintain 150°C to remove unreacted alcohols and then sparged for about 30 min with steam at 3.15 kg/cm$^2$ absolute. The reaction mixture was cooled and analyzed using gas chromatography as:

42.9% di(n-hexyl) adipate
12.1% di(cyclohexyl) adipate
45.0% n-hexyl, cyclohexyl adipate.

From the above adipate analysis, the reacted NHA/CHA weight ratio was 65.7/34.3.

Though the compositions of the invention are preferably used to plasticize PVB resin, they may alternatively be similarly usable to plasticize natural rubber, styrene-butadiene rubber and the following

polymers: polyvinyl chloride, cellulose nitrate, cellulose acetate and cellulose acetate-butyrate.

The preceding description is for illustration only and is not to be taken in a limited sense. Various modifications and alterations will be readily suggested to persons skilled in the art. It is intended, therefore, that the foregoing be considered as exemplary only and that the scope of the invention be ascertained from the scope of the following claims.

## Claims

**1.** A composition of matter comprising a mixture of pure and mixed adipates made from: (a) n-hexanol; and (b) cyclohexanol; wherein the proportions of (a) and (b) in parts by weight per 100 parts of (a) and (b) are:
(a) 50-70
(b) 50-30.

**2.** The composition of claim 1 wherein said proportions are:
(a) 60-70
(b) 40-30.

**3.** The composition of claim 1 or 2 wherein the proportion is:
(a) 65
(b) 35.

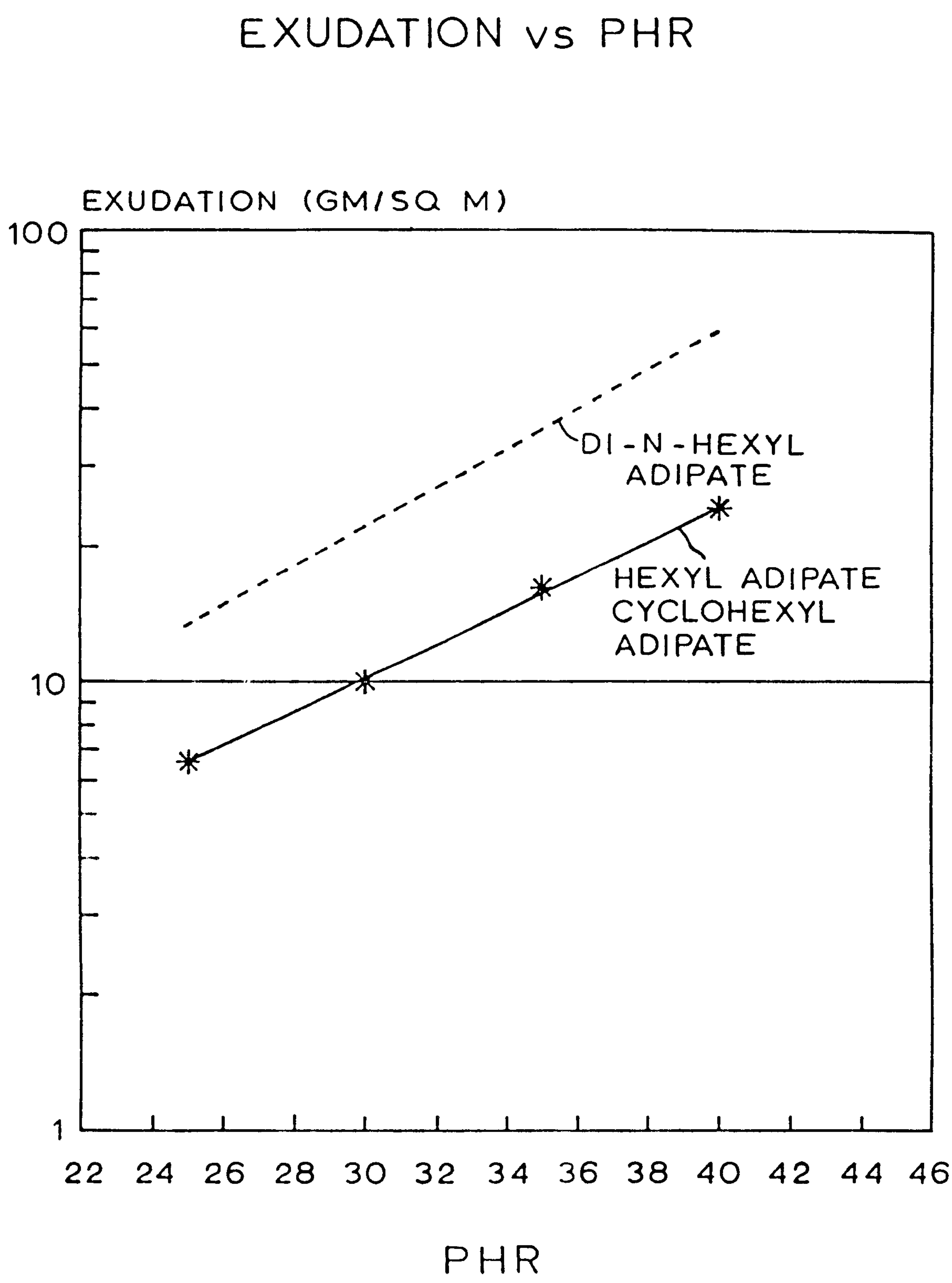
EXUDATION vs PHR
EXUDATION (GM/SQ M)
100
10
1
22
24
26
28
30
32
34
36
38
40
42
44
46
PHR
DI-N-HEXYL ADIPATE
HEXYL ADIPATE CYCLOHEXYL ADIPATE

FIG. 1

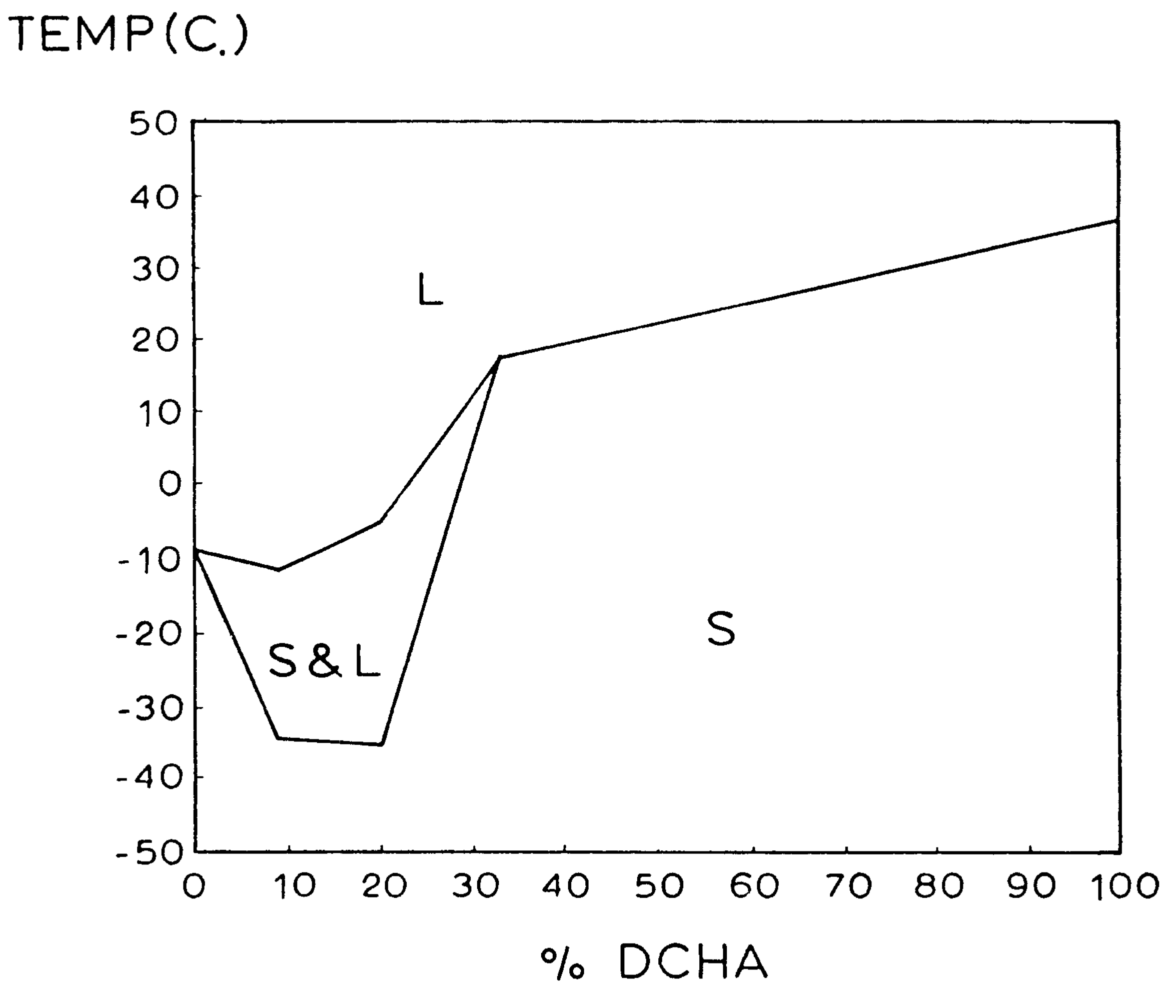
TEMP(C.)
50
40
30
20
10
0
-10
-20
-30
-40
-50
L
S&L
S
0
10
20
30
40
50
60
70
80
90
100
% DCHA

FIG. 2

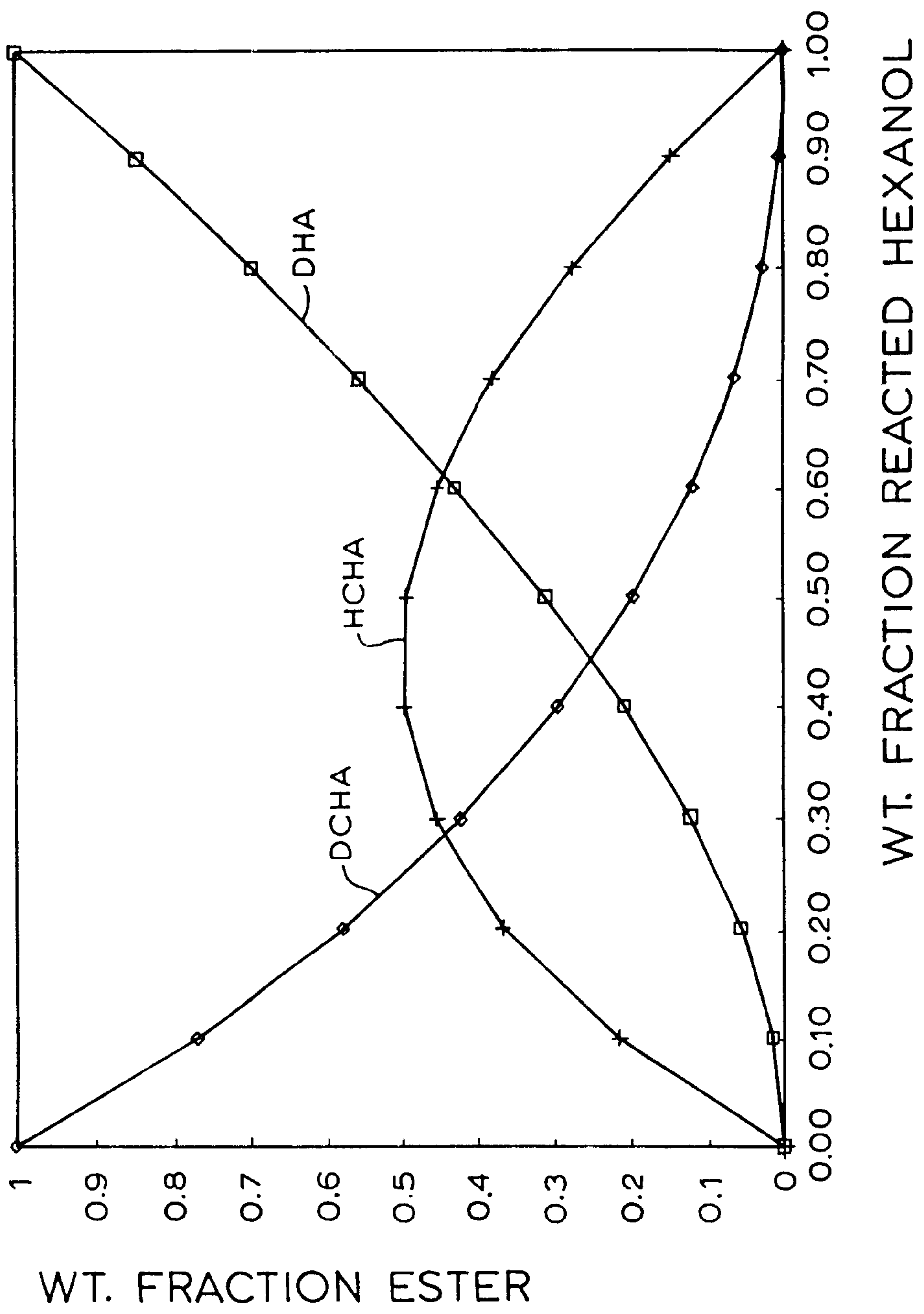
WT. FRACTION ESTER
1
0.9
0.8
0.7
0.6
0.5
0.4
0.3
0.2
0.1
0
0.00
0.10
0.20
0.30
0.40
0.50
0.60
0.70
0.80
0.90
1.00
WT. FRACTION REACTED HEXANOL
DHA
HCHA
DCHA

FIG. 3